# EUROPEAN PATENT SPECIFICATION

(11) **EP 1 829 978 B1**
(45) Date of publication and mention of the grant of the patent: **21.09.2011**
(21) Application number: 05814218.3
(22) Date of filing: 09.12.2005
(51) Int. Cl.: C12Q 1/68, C12N 15/09

(54) **METHOD OF DETECTING GENE METHYLATION AND METHOD OF EXAMINING NEOPLASM BY DETECTING METHYLATION**
VERFAHREN ZUM NACHWEIS VON GENMETHYLIERUNG UND VERFAHREN ZUR UNTERSUCHUNG VON NEOPLASMA MITTELS METHYLIERUNGSNACHWEIS
PROCEDE CONSISTANT A DETECTER LA METHYLATION D'UN GENE ET PROCEDE CONSISTANT A RECHERCHER UN NEOPLASME EN DETECTANT LA METHYLATION

(30) Priority: 13.12.2004 JP 2004359471; 13.12.2004 JP 2004360339
(43) Date of publication of application: 05.09.2007
(73) Proprietor: Bio-Dixam LLC, Okayama-shi Okayama 700-0927 (JP)
(72) Inventor: NAGASAKA, Takeshi, Hekinan-shi, Aichi 4470043 (JP); MATSUBARA, Nagahide, Okayama-shi, Okayama 7000927 (JP); TANAKA, Noriaki, Asakuchi-gun, Okayama 7190252 (JP)
(74) Representative: Zwicker, Jörk
(86) International application number: PCT/JP2005/022671
(87) International publication number: WO 2006/064737

(56) References cited:
- EP-A- 0 620 282
- EP-A- 1 394 172
- WO-A-97/46705
- WO-A2-02/103320
- JP-A- 7 236 499
- JP-A- 2004 527 245
- US-A1- 2002 119 478
- ISHIZAWA M ET AL: "SIMPLE PROCEDURE OF DNA ISOLATION FROM HUMAN SERUM" NUCLEIC ACIDS RESEARCH, OXFORD UNIVERSITY PRESS, SURREY, GB, vol. 19, no. 20, 1 January 1991 (1991-01-01), page 5792, XP001093804 ISSN: 0305-1048
- MORRIS D: "Hydrolysis of starch and glycogen by blood amylase", THE JOURNAL OF BIOLOGICAL CHEMISTRY, vol. 148, May 1943 (1943-05), pages 271-273,
- SOMOGYI M: "Micromethods for the estimation of diastase", THE JOURNAL OF BIOLOGICAL CHEMISTRY, vol. 125, September 1938 (1938-09), pages 399-414,
- MONTEIRO L ET AL: "Complex polysaccharides as PCR inhibitors in feces: Helicobacter pylori model", JOURNAL OF CLINICAL MICROBIOLOGY, vol. 35, no. 4, April 1997 (1997-04), pages 995-998, XP000867220,

## Description

### Technical Field

The present disclosure relates to a method for pre-treating a biological sample in gene analysis method for detecting methylation in genes and/or gene loci contained in the biological sample, wherein the biological sample is supplied with a polysaccharide and without protein denaturing agent added thereto. The present invention also relates to a method for detecting methylation in genes and/or gene loci contained in the pre-treated biological sample. The present invention further relates to a neoplasm examination method wherein the method for detecting methylation is used to detect methylation in specific genes.

This application claims priority of Japanese Patent Application No. 2004-359471 and Japanese Patent Application No. 2004-360339.

### Background Art

Diagnosis of infectious diseases and gene diagnosis have now become accessible through analysis of genes present in biological samples. For example, viruses, an enteric bacterial flora, pathogenic bacteria, gastrointestinal mucosa cells, malignant neoplasm cells, or neoplasm cells present in bloods or feces of mammals, and gene segments present in secreted fluids can be used to investigate pathological causes of various disorders and diagnose them, which is extremely useful for confirming health condition for an individual and early detection of malignant neoplasm.

In recent years, there has been reported that gene is frequently recognized to acquire aberrant methylation as one of features of neoplasm and malignant neoplasm.
In eukaryote cells, cytosine residue present immediately at the 5' side of a guanosine is methylated preferentially in a CG -deficient region (Bird, A., Nature (1986) 321: 209). In the meantime, a region referred to as a CpG region, which comprises a discontinuous CG dinucleotide, is present in the promoter region of a gene, but in an autosomal chromosome, almost all gene-related CpG regions are protected from methylation. Methylation over a broad range of CpG region is associated with inactivation of imprinted-gene transcription and inactivation of gene transcription on a female inactivated X-chromosome. Recent studies report that aberrant methylation in human tumors occurs in the promoter regions of various genes (Non-patent Documents 1 to 3).

Methylated cytosine in a CpG region was conventionally detected using a methylation sensitive restriction enzyme or a methylation reactive chemical substance. But, this method is disadvantageously unsuitable for a wide range of applications because it can analyze nothing but a limited region with a methylation possible site contained. Furthermore, the method can analyze nothing but a CpG region which the restriction enzyme can recognize in a sequence. Even a method wherein the methylation sensitive restriction enzyme is combined with a nucleic acid amplification reaction is not reliable for a case where only a very small part of contained methylation allele is usable to analyze, e.g., a case where high-methylation in a suppressor oncogene must be detected in a small amount of sample, because the method is difficult to distinguish an incomplete cleavage by the restriction enzyme from a small number in methylation allele.

As a method for effectively detecting methylation in genes without using the above-mentioned methylation sensitive restriction enzyme or the methylation reactive chemical substance, the method for processing genes by a bisulfite is mentioned. Genes are processed with a bisulfite to convert all unmethylated cytosines to uracils to give a modified nucleic acid, which then can be used to detect methylation in genes. As such method, methylation specific PCR (MSP) method (Patent Document 1), COBRA (Combined Bisulfite Restriction Assay) (Non-patent Document 4), Methylight method (Patent Document 2) are mentioned. However, these methods require extraction and purification of DNAs before bisulfite processing and also need relatively large amount of DNAs. In addition, the bisulfite-processed DNAs must be purified again before they are amplified by PCR to analysis.

For extraction and purification of DNA, an organic solvent such as phenol or chloroform is normally used, and many commercial kits are available on the market. As a method which does not use an organic solvent, MagExtractor (Toyobo) and QIAamp Stool DNA Isolation Kit (manufactured by QIAGEN) are commercially available. The former crushes membrane with a bead to adsorb DNAs, which are then collected by a magnetic bead. The latter heats and denatures a membrane protein to isolate DNAs.

As a reagent kit capable of extracting and purifying DNAs without the use of organic solvents, the kit which is a combination of a proteolytic enzyme, an aqueous solution containing at least one of protein denaturing agent, a surface active agent, a chelating agent and protein denaturing agent, and a salt and a coprecipitating agent, and denatured protein dissolving agent (Patent Document 3), and a kit which includes protein denaturing agent, a coprecipitating agent, and protein denaturing agent (Patent Document 4) are disclosed. These kits contain a proteolytic enzyme and protein denaturing agent as indispensable components and provide a nucleic acid enough purified to use in PCR.

As mentioned above, detection of a methylated DNA generally can not be exempted from a cumbersome procedure and a long time, because the nucleic acid must be artificially modified and repeatedly purified before amplified to analyze. Conventionally, the nucleic acid to modify is extracted and purified in the same method to give a highly purified DNA as in purification to amplify.

As a method for using feces to detect a malignant neoplasm in a digestive organ (particularly a large intestinal cancer), the FOBT (Fecal Occult-Blood Testing) is mentioned. Three randomized controlled studies performed in EU and US could use the fecal occult-blood assay to detect early stage cancers, and, as the result, succeeded in reduction of mortality rate (15 to 35% reduction in the medical examination group). Further, early stage cancers in the medical examination group could be detected to lead to a recognized reduction in morbidity rate and infiltrating cancer (N Eng J Med (1993) 328:1365-71, Lancet (1996) 348:1472-7, Lancet (1996) 348:1467-71). However, this examination method is low in sensitivity, and can only detect the large intestinal cancers of the fecal occult-blood reaction positive patients at a rate as low as 2 to 17%. Further, only 27% of patients with large intestinal cancer in the three randomized controlled studies performed in EU and US could be identified to have large intestinal cancer by the fecal occult-blood reaction, indicating that the method is not enough to satisfy.

Recent development of molecular biology has cleared gene mutation in relation with neoplasm and malignant neoplasm in a digestive organ. As for the mutation of gene in a large intestinal cancer, for example, point mutation in KRAS gene, point mutation in APC (adenomatous polyposis coil) gene, point mutation in p53 gene, and point mutation in BRAF gene have been reported. However, all these gene mutations are not always detected to cover all large intestinal cancers to examine, and in many cases, the detection methods and technologies involved are still difficult to perform and take high costs. It is presumed that, if mutation of gene could be detected from feces, it would allow identification of neoplasm and malignant neoplasm (Nat Rev Cancer. (2002) Mar; 2 (3) :210-9) . Further, one report (Non-patent Document 5) describes an attempted method to diagnose large intestinal cancers wherein human feces are heated to denature the membrane protein, and then a DNA-isolating kit is used to extract and purify a DNA, which is then processed with bisulfite to detect an aberrant methylation characteristic to neoplasm. This method uses a kit for extraction and purification of the DNA, but is not economical.
[Non-patent Document 1] Jones, P.A. et al., Nat. Genet., (1999); 21: 163-167
[Non-patent Document 2] Issa et al. , Ann. N.Y. Acad. Sci., (2000); 910: 140-153
[Non-patent Document 3] Herman et al., N. Engl. J. Med., (2003); 349:2042-2054
[Non-patent Document 4] Xiong et al., Nucleic Acids Res (1997); 25:2532-2534
[Non-patent Document 5] Muller et al., The Lancet, (2004) ; 363:1283-1285
[Patent Document 1] WO2000-511776 A
[Patent Document 2] W02002-543852 A
[Patent Document 3] JP H07-236499 A
[Patent Document 4] JP 2001-17173 A

US 2002/119478 A1 (Umansky Samuil R et al.) (2002-08-29) anticipates a method for detecting methylation in genes in which DNA is extracted using a method which involves the use of glycogen as a carrier and guanidine (iso)thiocyanate as a protein denaturing agent.

### Disclosure of the Invention

### Problems to be Solved by the Invention

An object of the present invention is to provide a method for detecting methylation in genes contained in a biological sample in a simple and economical manner. Another object of the present invention is to provide a method for examining neoplasm in an accurate and sensitive manner using the method for detecting methylation in genes. Means for Solving the Problems

The present inventors made an intensive study to solve above-mentioned problems, and have found that a sample supplied with a polysaccharide and without protein denaturing agent can give a DNA which, even if not purified, is processed with a bisulfite to allow detection of methylated cytosine that has not been modified to convert to uracil, because unmethylated cytosine is processed with the bisulfite to convert to uracil, thereby to distinguish methylated cytosine from unmethylated cytosine. The finding has completed the present invention. Furthermore, it has been found that several types of methylations in genes contained in a biological sample are detected to calculate their total value, allowing more accurate examination of a neoplasm. The finding also has completed the present invention.

Namely, the present invention is defined in and by the claims. Also disclosed is:
1. A method for pre-treating a biological sample, wherein the biological sample is supplied with a polysaccharide and without protein denaturing agent to carry out a method for detecting methylation in genes and/or gene loci contained in the biological sample.
2. The method for pre-treating the biological sample according to previous item 1, wherein the polysaccharide is glycogen.
3. A method for detecting methylation in genes and/or gene loci, wherein the biological sample pretreated by the method according to previous item 1 or 2 is contacted with a bisulfite to convert unmethylated cytosine into uracil in genes and/or gene loci contained in the biological sample, thereby to detect cytosine which has not been converted into uracil.
4. A method for examining a neoplasm, wherein methylation in genes and/or gene loci of the SFRP2 gene, the DCC gene and the MGMT gene contained in a biological sample is detected to calculate the total of their respective methylation values.
5. The method for examining a neoplasm according to previous item 4, wherein methylation on the APC gene and/or the hMLH1 gene is further detected to calculate the total of their respective methylation values.
6. The method for examining a neoplasm according to previous item 4 or 5, wherein methylation is detected by the method according to previous item 3.

### Effect of the Invention

The pretreatment method of the present disclosure provides a sample which can be simply and in a short time treated to convert unmethylated cytosine into uracil at genes and/or gene loci contained in a biological sample. Further, the pretreatment method of the present disclosure is so simple to manipulate that it can prevent a sample from being contaminated or lost and provide a large amount of pretreated samples. Therefore, this method can be applied to an analysis method which a slight amount of sample is prepared for to analyze or needs a large amount of nucleic acid to analyze.
Furthermore, the examination method of the present invention can examine a neoplasm accurately and sensitively.

### Brief Description of the Drawings

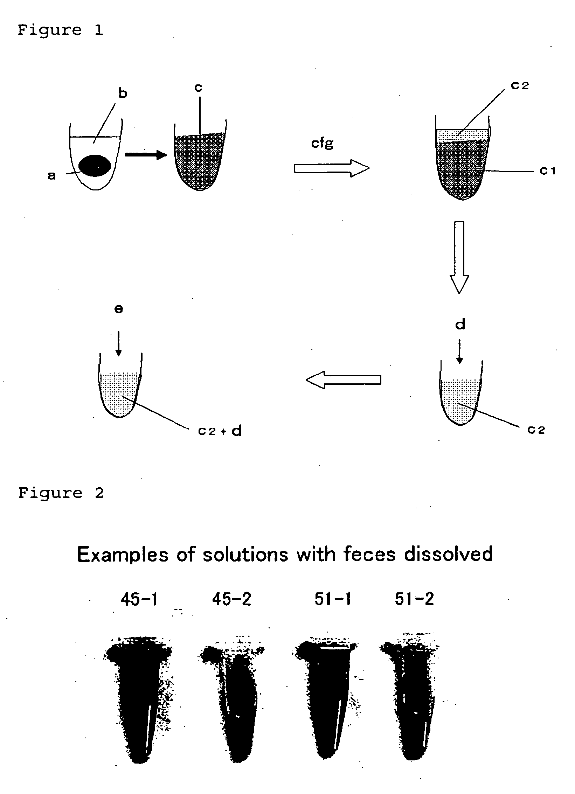

FIG. 1 is a drawing illustrating a series of manipulations comprising the pretreatment method for using feces as a biological sample (Example 1).
FIG. 2 is a drawing showing examples of solutions with feces dissolved (Example 1).

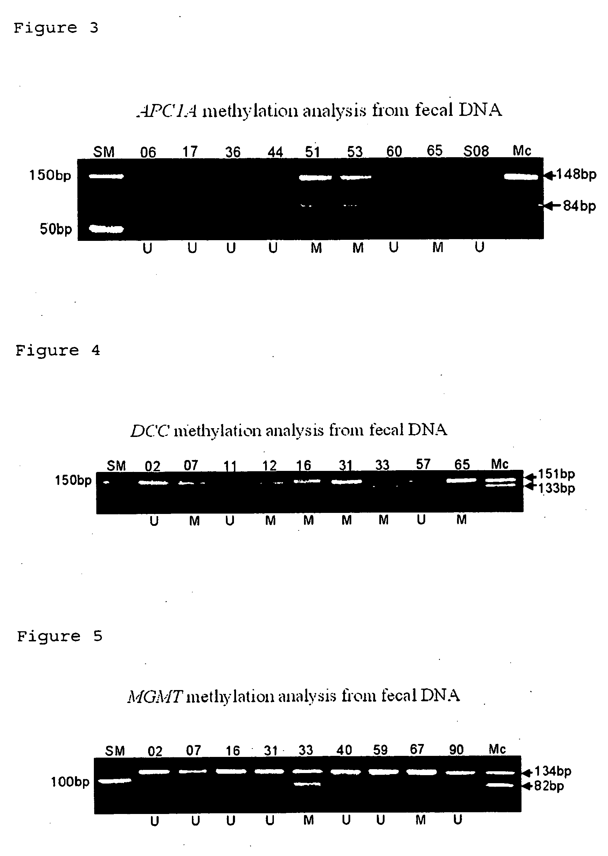

FIG. 3 is a drawing showing investigation result of presence or absence of methylation in 1A promoter region of APC gene in feces. SM denotes a size marker and numbers denote each a sample number. Further, Mc denotes a control of methylation, M denotes that methylation has been detected, U denotes that methylation has not been detected (Example 3).
FIG. 4 is a drawing showing investigation result of whether promoter region or 5' region of DCC gene in feces is methylated or not. SM denotes a size marker and numbers denote each a sample number. Further, Mc denotes a control of methylation, M denotes that methylation has been detected, U denotes that methylation has not been detected (Example 3).
FIG. 5 is a drawing showing investigation result of whether promoter region of MGMT gene in feces is methylated or not. SM denotes a size marker and numbers denote each a sample number. Further, Mc denotes a control of methylation, M denotes that methylation has been detected, U denotes that methylation has not been detected (Example 3).

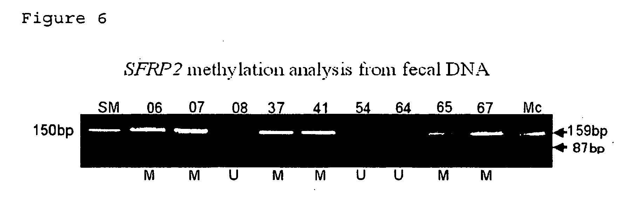

FIG. 6 is a drawing showing investigation result of whether promoter region or 5' region of SFRP2 gene in feces is methylated or not. SM denotes a size marker and numbers denote each a sample number. Further, Mc denotes a control of methylation, M denotes that methylation has been detected, U denotes that methylation has not been detected (Example 3).

### Description of Symbols

- a: Sample (feces)
- b: Dissolving buffer solution
- c: Sample solution
- c1: Precipitation in sample solution
- c2: Supernatant in sample solution
- d: Glycogen
- e: Sodium bisulfite

### Description of the Preferred Embodiment

In the present invention, "biological sample" includes a tissue sampled from a living body, and a body fluid such as blood, urine, serum, feces, ejaculated semen, expectorated sputum, nasal discharge, saliva, cerebral fluid, or tear fluid. As the biological sample of the present invention, feces are particularly preferable. In feces, enteric mucosa cell, blood cell, enteric bacterial flora, pathogenic microorganism (e.g., bacteria, virus), enteric neoplasm (including large intestinal cancer and polyp) are present. Feces are useful as a noninvasive DNA material for analysis of genes associated with various types of disorders and microorganisms, for example, causative gene of inherited disorders, gene derived from large intestinal cancer tissue, or gene derived from bacteria.

Preferably, the biological sample collected is at first suspended or dissolved in a suitable dissolving solution, and then diluted appropriately to produce a sample solution. The dissolving solution for suspending or dissolving the biological sample may be selected to use appropriately from known fluids without protein denaturing agent contained, taking account of type and amount of the biological sample. In the present invention, "protein denaturing agent" denotes protein denaturing agent used normally for extracting a nucleic acid such as DNA from a biological sample, and includes particularly urea, guanidine hydrochloric acid, guanidine sulfate, and guanidine thiocyanate. Meanwhile, the dissolving solution may contain in advance a nonspecific proteolytic enzyme such as proteinase K, pronase, and subtilisin.

The dissolving solution is not limited particularly as long as it can dissolve solid matters contained in feces as a biological sample and contains no protein denaturing agent, and preferably includes a Tris-EDTA buffer solution having a pH of around 9. 1 mg of feces can be supplied with 0.1 to 100 µL, preferably 1 to 30 µL, more preferably approximately 3 to 7 µL of the dissolving solution to prepare a sample solution. For example, 100 mg of feces is placed in a 1.5 mL tube and supplied with 500 µL of the dissolving solution to dissolve the feces.

In order to detect the gene in a microorganism such as an enteric bacterium in feces, a sample solution may be heated, for example, at 60 to 95°C for 10 min to 10 hours to denature the membrane proteins of the microorganism. The sample solution thus obtained may be centrifuged as required. The centrifugation may be performed at a speed of 3,000 to 8,000 rpm, preferably 4,000 to 6,000 rpm, more preferably approximately 5,000 rpm for 1 to 5 min. As the sample, feces are centrifuged to remove unnecessary solid matters or the like, and the supernatant solution is preferably used.

### (Pretreatment method)

The sample solution obtained as mentioned above or the supernatant obtained by centrifugation of the sample solution (hereinafter both are in some cases referred simply to as "sample solution") is subjected to a pretreatment before genes are modified. A polysaccharide is added for the pretreatment. The polysaccharide is not limited particularly as long as it functions as a DNA carrier and does not affect a bisulfite processing to perform later, and includes particularly glycogen as a preferable one.

The polysaccharide is added in such an amount that it may function as the DNA carrier. For example, 2 to 200 µg of glycogen may be added to 10 to 100 µL of the sample solution. More preferably, 45 µg of glycogen may be added to 42 µL of above-mentioned solution to make a total volume of 45 µL. Glycogen commercially available may be used. Although glycogen in powder may be added directly to the sample solution, a glycogen solution prepared is preferably added. The glycogen solution may have an appropriately adjusted concentration, for example of 15 to 20 mg/mL, depending on a sample amount or the like. The sample solution is incubated with the contained glycogen at 37°C for 15 to 30 min.

Next, DNAs in the sample solution are preferably subjected to an alkali denaturing reaction to convert the double-stranded DNA into the single-stranded DNA. The alkali denaturing reaction may be carried out by a known method. For example, it may be carried out by addition of a sodium hydroxide solution. The resultant solution may be further incubated at 37°C for 15 to 30 min. A commercially available kit for analysis, which comprises an alkali denaturing reagent, may be used to carry out the alkali denaturing reaction according to a method described in the instruction manual.

For example, 43 µL of the supernatant obtained by centrifugation of the sample solution with dissolved feces is supplied with 2 µL of a glycogen solution (15 to 20 mg/mL) and 5 µL of a sodium hydroxide solution (M-Dilution Buffer ^{™} when DNA methylation Kit ^{™} (ZYMO RESEARCH) is used) to make a total volume of 50 µL. In this case, the resultant solution supplied with the glycogen solution and the sodium hydroxide solution may be incubated once at 35 to 40°C, preferably 37°C for 15 to 30 min.

### (Detection method for methylation)

The present invention also covers a method for detecting methylation in genes and/or gene loci. In the present invention, "detecting methylation in genes and/or gene loci" denotes detecting methylation of cytosine in a CpG island/CpG array associated with a specific gene promoter region or a 5' region or a gene locus present in a biological sample. For detection, a bisulfite is used to modify all unmethylated cytosines in genes present in the biological sample to convert into uracils. Methylated cytosine, which can not be modified with the bisulfite, is not converted into uracil. Therefore, a target is subjected to the modification processing and then detected to be cytosine, indicating that cytosine has been methylated. The indication can lead to a judgment that genes and/or gene loci containing cytosine also have been methylated.

A known method can be applied to the sample solution pretreated as above-mentioned to modify unmethylated cytosine. For example, a commercially available modification reagent containing bisulfite can be directly contacted to the sample solution pretreated to modify unmethylated cytosine in genes and/or gene loci contained in the sample solution, thereby to convert into uracil. In particular, a commercially available kit for DNA methylation detection, such as, DNA methylation Kit^{™} (ZYMO RESEARCH), MethylEasy^{™} (Human Genetic Signature), and CpGenome DNA Modification Kit ^{™} (CHEMICON) can be used.

For detection of methylation, a per se known method may be used. For detection of presence or absence of methylation in genes, methylation specific PCR (MSP) method, COBRA (combined bisulfite restriction assay) method, Methylight method or the like may be used. For example, in order to confirm whether unmethylated cytosine in a PCR-amplified DNA has been converted to uracil or not, both one primer which can amplify the sequence with unmethylated cytosine converted to uracil and another primer which can amplify the sequence without methylated cytosine converted to uracil may be used to amplify the nucleic acid, thereby to get an amplified product to confirm. From viewpoint of characteristics of the sample, a gene to amplify has preferably a length of 200 bp or less, more preferably 180 bp or less.

### (Method for examining neoplasm)

The present invention also covers examination of a neoplasm by detecting methylation in genes and/or gene loci contained in the biological sample. In the present invention, genes contained in the biological sample, specifically, includes SFRP2 (secreted apoptosis-related protein-2) gene, DCC (deleted in colorectal carcinomas) gene, and MGMT (06-Mehylguanine-DNA methyltransferase) gene. Furthermore, APC (adenomatous polyposis coli) gene and/or hMLH1 gene may be included. These genes and/or gene loci are known particularly as genes associated with malignant neoplasm (cancer) and neoplasm (adenoma) of digestive organs. By detecting methylation in each of the genes and/or gene loci mentioned above, it is possible to examine neoplasm associated with digestive organs.

The neoplasm may be a malignant neoplasm (cancer) or a neoplasm (adenoma). In this case, a biological sample such as feces, before processed with a bisulfite to detect methylation, may be pretreated by any method, for example, conveniently and preferably by the pretreatment method of the present disclosure described above wherein the sample is supplied with a polysaccharide and without protein denaturing agent. For example, commercially available MagExtractor (Toyobo) and QIAamp Stool DNA Isolation Kit (manufactured by QIAGEN) may be used to extract a DNA by a per se known method.

"Calculate a total of methylated genes' respective methylation values" in the present invention may be to calculate a quantitative total of methylation values detected quantitatively in the gene promoter region or in the 5' region of each genes detected through detection of methylation in above-mentioned genes and/or gene loci, or to calculate a total in number of methylated genes by a method accompanied with no quantitative determination. For example, neoplasm in digestive organs can be examined accurately, for example, by obtaining a total value of methylation in SFRP2, DCC and MGMT genes. Moreover, a total value of APC and/or hMLH1 genes may be included.

In particular, methylation of cytosine in the CpG region present in the promoter region or the 5' region of SFRP2 gene, methylation of cytosine in the CpG region present in the promoter region or the 5' region of DDC gene, and methylation of cytosine in the CpG region present in the promoter region or the 5' region of MGMT gene, are detected to calculate a total of their respective methylation values. Furthermore, methylation of cytosine in the CpG region present in the 1A promoter region of APC gene and/or methylation of cytosine in the CpG region present in the promoter region or the 5' region of DDC gene are detected to calculate a total of their respective methylation values, which may be added to the total of the methylation values derived from three kinds of genes shown previously, thereby to get a wholly total value. Thus, neoplasm present in a digestive organ can be detected.

The base sequence of specific genes or gene loci in the present invention is shown in NC_000004 for SFRP2 gene, in NT_033905.3 for DDC gene, in HSU95038 for MGMT gene, in Genebank Accession No. HSU02509 for APC gene, and in Genebank Accession No. AB017806 for hMLH1 gene. Further, methylation of SFRP1 gene may be investigated, wherein the base sequence is shown in NC_000008 for the SFRP1 gene.
As primers for amplifying a nucleic acid in the CpG region present in the promoter region or the 5' region in each of these genes, the followings may be used:

Primers capable of amplifying the CpG region present in the 1A promoter region of APC gene are as follows:
APC1A-NF: 5'-ATATTTTYGAGGGGTAYGGGGTTA (SEQ ID NO: 1)
APC1A-MF: 5'-TATTGCGGAGTGCGGGTC (SEQ ID NO: 2)
APC1A-NR: 5'-ACRAAAATAAAAAACRCCCTAATC (SEQ ID NO: 3).

APC1A-NF (SEQ ID NO: 1) and APC1A-NR (SEQ ID NO: 3) are designed to hybridize to both one allele with methylated cytosine contained and another allele with unmethylated cytosine contained, and these two primers provide an amplification product of 148 bp whether an allele contains methylated cytosine or not.
In the meantime, APC1A-MF (SEQ ID NO: 2) is designed to hybridize to only one allele with methylated cytosine contained, and the two primers of APClA-MS (SEQ ID NO: 2) and APClA-NAS (SEQ ID NO: 3) provide an amplification product of 84 bp if an allele contains methylated cytosine.

Namely, cytosine in the CpG region present in the 1A promoter region of APC gene, which is recognized to have been methylated, indicates that the two gene amplification products of 148 bp and 84 bp are recognized, while cytosine in the CpG region present in the 1A promoter region of APC gene, which is not recognized to have been methylated, indicates that only one gene amplification product of 148 bp is recognized (FIG. 1).
The primers are not limited to above-mentioned primers as long as they are capable of amplifying a region for APC gene to require, and primers with similar functions may be used. Preferably, the primers shown by SEQ ID NOS: 1 to 3 can be used in one set to confirm amplification of different DNAs in base number depending on methylated cytosine in the CpG region present in the promoter region of APC gene after one-time nucleic acid amplification.

Primers capable of amplifying the CpG region present in the promoter region or the 5' region of DDC gene are as follows:
DCC-NF: 5'-AGGTGGAGAAAGAGGTGGAGGAA (SEQ ID NO: 4)
DCC-MR: 5'-ACCAAAAATCGCGAACAACG (SEQ ID NO: 5)
DCC-NR: 5' -TCAACCAACACCTTCRAAACCAAA (SEQ ID NO: 6).

DCC-NF (SEQ ID NO: 4) and DCC-NR (SEQ ID NO: 6) are designed to hybridize to both one allele with methylated cytosine contained and another allele with unmethylated cytosine contained, and a set of these two primers provides an amplification product of 151 bp whether an allele contains methylated cytosine or not.
Meanwhile, DCC-MR (SEQ ID NO: 5) is designed to hybridize to only one allele with methylated cytosine contained, and the two primers of DDC-MR (SEQ ID NO: 5) and DCC-NF (SEQ ID NO: 4) provide an amplification product of 133 bp if an allele contains methylated cytosine.

Namely, cytosine in the CpG region present in the promoter region or the 5' region of DCC gene, which is recognized to have been methylated, indicates that the two gene amplification products of 151 bp and 133 bp are recognized, while cytosine in the CpG region present in the promoter region or the 5' region of DCC gene, which is not recognized to have been methylated, indicates that only one gene amplification product of 151 bp is recognized (FIG. 2).
The primers are not limited to above-mentioned primers as long as they are capable of amplifying a region for DDC gene to require, and primers with similar functions may be used. Preferably, the primers shown by SEQ ID NOS: 4 to 6 can be used in one set to confirm amplification of different DNAs in base number depending on methylated cytosine in the CpG region present in the promoter region or the 5' region of DDC gene after one-time nucleic acid amplification.

Primers capable of amplifying the CpG region present in the promoter region of MGMT gene are as follows:
MGMT3-NF: 5'-GYGTTTYGGATATGTTGG GAT (SEQ ID NO: 7)
MGMT3-MF: 5'-ACGTTGGTAGGTTTTCGC (SEQ ID NO: 8)
MGMT3-NR: 5'-AACTCCRCACTCTTCCRAAAACRA (SEQ ID NO: 9).

MGMT3-NF (SEQ ID NO: 7) and MGMT-3-NR (SEQ ID NO: 9) are designed to hybridize to both one allele with methylated cytosine contained and another allele with unmethylated cytosine contained, and a set of these two primers provides an amplification product of 134 bp whether an allele contains methylated cytosine or not.
Meanwhile, MGMT3-MF (SEQ ID NO: 8) is designed to hybridize to only one allele with methylated cytosine contained, and the two primers of MGMT3-MF (SEQ ID NO: 8) and MGMT3-NR (SEQ ID NO: 9) provide an amplification product of 82 bp if an allele contains methylated cytosine.

Namely, cytosine in the CpG region present in the promoter region of MGMT gene, which is recognized to have been methylated, indicates that the two gene amplification products of 134 bp and 82 bp are recognized, while cytosine in the CpG region present in the promoter region of MGMT gene, which is not recognized to have been methylated, indicates that only one gene amplification product of 134 bp is recognized (FIG. 3).
The primers are not limited to above-mentioned primers as long as they are capable of amplifying a region for MGMT gene to require, and primers with similar functions may be used. Preferably, the primers shown by SEQ ID NOS: 7 to 9 can be used in one set to confirm amplification of different DNAs in base number depending on methylated cytosine in the CpG region present in the promoter region of MGMT gene after one-time nucleic acid amplification.

Primers capable of amplifying CpG region present in promoter region of hMLH1 gene are as follows:
hMLH15-NF: 5'-YGGGTAAGTYGTTTTGAYGTAGA (SEQ ID NO: 10)
hMLH15-MF: 5'-CGTTCGTCGTTCGTTATATATC (SEQ ID NO: 11)
hMLH15-NR: 5'-TATACCTAATCTATCRCCRCCTCA (SEQ ID NO: 12).

hMLH15-NF (SEQ ID NO: 10) and hMLH15-NR (SEQ ID NO: 12) are designed to hybridize to both one allele with methylated cytosine contained and another allele with unmethylated cytosine contained, and a set of these two primers provides an amplification product of 149 bp whether an allele contains methylated cytosine or not.
Meanwhile, hMLH15-MF (SEQ ID NO: 11) is designed to hybridize to only one allele with methylated cytosine contained, and the two primer sets of hMLH15-MF (SEQ ID NO: 11) and hMLH15-NR (SEQ ID NO: 12) provide an amplification product of 109 bp if an allele contains methylated cytosine.

Namely, cytosine in the CpG region present in the promoter region of hMLH1 gene, which is recognized to have been methylated, indicates that the two gene amplification products of 149 bp and 109 bp are recognized, while cytosine in the CpG region present in the promoter region of hMLH1 gene, which is not recognized to have been methylated, indicates that only one gene amplification product of 149 bp is recognized
The primers are not limited to above-mentioned primers as long as they are capable of amplifying a region for hMLH1 gene to require, and primers with similar functions may be used. The primers shown by SEQ ID NOS: 10 to 12 can be used in one set to confirm amplification of different DNAs in base number depending on methylated cytosine in the CpG region present in the promoter region of hMLH1 gene after one-time nucleic acid amplification.

Primers capable of amplifying the CpG region present in the promoter region or the 5' region of SFRP1 gene are as follows:
S1-NF:5'-GYGTTTTTTTGTTYGTYGTATTTT (SEQ ID NO: 13)
S1-MF:5'-TCGTAGCGTCGTTTTTTC (SEQ ID NO: 14)
S1-NR:5' -AAAACRCAATCCCCAACRTTAC (SEQ ID NO: 15).

S1-NF (SEQ ID NO: 13) and S1-NR (SEQ ID NO: 15) are designed to hybridize to both one allele with methylated cytosine contained and another allele with unmethylated cytosine contained, and a set of these two primers provides an amplification product of 166 bp whether an allele contains methylated cytosine or not.
Meanwhile, S1-MF (SEQ ID NO: 14) is designed to hybridize to only one allele with methylated cytosine contained, and the two primers of S1-MF (SEQ ID NO: 14) and S1-NR (SEQ ID NO: 15) provide an amplification product of 120 bp if an allele contains methylated cytosine.

Namely, cytosine in the CpG region present in the promoter region or the 5' region of SFRP1 gene, which is recognized to have been methylated, indicates that the two gene amplification products of 166 bp and 120 bp are recognized, while cytosine in the CpG region present in the promoter region or the 5' region of SFRP1 gene, which is not recognized to have been methylated, indicates that only one gene amplification product of 166 bp is recognized.
The primers are not limited to above-mentioned primers as long as they are capable of amplifying a region for SFRP1 gene to require, and primers with similar functions may be used. Preferably, the primers shown by SEQ ID NOS: 13 to 15 can be used in one set to confirm amplification of different DNAs in base number depending on methylated cytosine in the CpG region present in the promoter region or the 5' region of SFRP1 gene after one-time nucleic acid amplification.

Primers capable of amplifying CpG region present in promoter region or 5' region of SFRP2 gene are as follows:
S2-NF: 5'-GGTTGTTAGTTTTTYGGGGTTT (SEQ ID NO: 16)
S2-MF: 5'-TCGTTTCGTTTTTTTTCGGTTTC (SEQ ID NO: 17)
S2-NR: 5'-CAACAACAACRAACCAAAACCCTAC (SEQ ID NO: 18).

S2-NF (SEQ ID NO: 16) and S2-NR (SEQ ID NO: 18) are designed to hybridize to both one allele with methylated cytosine contained and another allele with unmethylated cytosine contained, and a set of these two primers provides an amplification product of 159 bp whether an allele contains methylated cytosine or not.
Meanwhile, S2-MF (SEQ ID NO: 17) is designed to hybridize only one allele with methylated cytosine contained, and the two primers of S2-MF (SEQ ID NO: 17) and S2-NR (SEQ ID NO: 18) provide an amplification product of 87 bp if an allele contains methylated cytosine.

Namely, cytosine in the CpG region present in the promoter region or the 5' region of SFRP2 gene, which is recognized to have been methylated, indicates that the two gene amplification products of 159 bp and 87 bp are recognized, while cytosine in the CpG region present in the promoter region or the 5' region of SFRP2 gene, which is not recognized to have been methylated, indicates that only one gene amplification product of 159 bp is recognized (FIG. 4).
The primers are not limited to above-mentioned primers as long as they are capable of amplifying a region for SFRP2 gene to require, and primers with similar functions may be used. Preferably, the primers shown by SEQ ID NOS: 16 to 18 can be used in one set to confirm amplification of different DNAs in base number depending on methylated cytosine in the CpG region present in the promoter region or the 5' region of SFRP2 gene after one-time nucleic acid amplification.

The amplification products can be confirmed by a per se known method. In particular, they can be confirmed by electrophoresis on agarose gel. Further, in order to amplify a DNA containing a target gene or gene locus in which to detect methylated cytosine, only a primer which is designed to hybridize to both one allele with methylated cytosine contained and another allele with unmethylated cytosine is used to amplify, thereby to give an amplification product. This amplification product can be confirmed by hybridization by DNA chip, clone library method, denaturing gradient gel electrophoresis method (DGGE method), temperature gradient gel electrophoresis method (TGGE method), Methylight method or SSCP method.

### (Reagent and kit)

The present disclosure also covers a sample pretreatment reagent which is used to pre-treat a sample before modification of a nucleic acid and comprises glycogen and without protein denaturing agent; a sample pretreatment reagent kit which is used for above-mentioned pretreatment method and comprises glycogen and without protein denaturing agent; and a nucleic acid modification reagent kit which is used for above-mentioned nucleic acid modification method.

Further, the present disclosure covers, in addition to a method for examining a neoplasm present in digestive organs, a primer and a primer set as above-mentioned. Furthermore, the present disclosure covers a reagent for examining and a reagent kit for detecting a neoplasm present in digestive organs. The present invention can use each of above-mentioned primers as a reagent, and the reagent kit may comprise the primers or the primer set. The reagent kit may comprise, in addition to above-mentioned primers, above-mentioned sample pretreatment reagent and a reagent for amplifying a nucleic acid, and a reagent to use for confirmation of amplified products.

### [Examples]

The present invention will be described hereinafter referring to Examples. It is apparent that the present invention is not limited thereto.

### (Example 1) A method for pretreating biological samples of feces

Pretreatment of the biological samples wherein the samples are feces is described referring to FIG. 1 and FIG. 2.
1) For a solution to dissolve feces, the solution (dissolution buffer solution) having a composition of 500 mmol/L Tris-HCl, 16 mmol/L EDTA, 10 mmol/L NaCl, pH 9.0 was prepared. To a 1.5 mL tube were added 78 mg of sample no. 9 feces, 117 mg of sample no. 12 feces, 162 mg of sample no. 45 feces, and 146 mg of sample no. 51 feces, respectively. Each of feces was dissolved in 1, 000 µL of dissolution buffer solution to prepare each of feces solution. Next, each of feces solution was heated at 95°C for 10 min (FIG. 1, upper left illustration).

### 2) Centrifugation of feces solution

Feces solution thus obtained was centrifuged at 5,000 rpm for 3 min to separate a supernatant and a precipitation (FIG. 1, upper right illustration).

### 3) Addition of glycogen to solution

2 µL of glycogen solution (15 mg/mL) was added to 43 µL of the supernatant of above-mentioned feces solutions (sample no. 45-1, 51-1) and stirred. 100 µL of the supernatant was diluted with 500 µL of the dissolution buffer solution (sample no. 45-2, 51-2) (FIG. 2), and then 2 µL of glycogen solution (15 mg/mL) was added to 43 µL of the diluted solution obtained, and stirred (FIG. 1, lower right illustration).

### 4) Denaturing of DNA to single strand

Steps described below were performed according to the procedures of DNA methylation kit (EZ: manufactured by ZYMO RESEARCH). 5 µL of M-Dilution Buffer (sodium hydroxide solution) included in the kit was added to each sample and incubated at 37°C for 15 min.

### 5) Conversion of unmethylated cytosine to uracil

Using DNA methylation detection kit DNA methylation kit^{™} (ZYMO RESEARCH), unmethylated cytosine was converted to uracil. CT Conversion reagent (sodium bisulfite solution) included in the kit was added to solutions of above-mentioned sample numbers 9, 12, 45-1, 51-1, 45-2, 51-2, and incubated to modify all unmethylated cytosines to convert to uracils (FIG. 1, lower left illustration).

### 6) Purification of DNA

Using Zymo-Spin I Column included in the kit, DNAs derived from modified gene thus obtained were purified to attain their high purities.

### (Example 2) Preliminary investigation

From 250 cases of patients with large intestinal cancer, 10 cases of patients with large intestine adenoma, and 85 cases of patients with stomach cancer, their respective neoplasm portions or normal mucosa portions were collected as biological samples, and the following investigations were performed.
Large intestine cancer tissues (including hereditary non-adenomatous large intestine cancer) from 250 cases who underwent surgical resection and endoscopical excision, 10 cases of large intestine adenomatous tissues (including familial large intestine adenomatosis), 85 cases of stomach cancer tissues, 225 cases of normal large intestine mucosal tissues, and 85 cases of normal stomach mucosa tissues were used as the biological samples to extract their respective DNAs, which were processed with sodium bisulfite. At first, 239 cases of large intestine cancer were used to investigate methylation in totally 15 types of gene promoter regions or 5' regions and gene loci (SFRP1, SFRP2, DCC, APC, MGMT, hMLH1, MINT1, MINT2, MINT31, CACNA1G, p14 , p16, THBS1, DAPK, COX2). From the results obtained, there were selected six genes (SFRP1, SFRP2, APC, DCC, MGMT, hMLH1) with higher frequency of methylation as the markers for detecting large intestine cancer and neoplasm of digestive organs.

Next, 250 cases of large intestine cancer tissues (including hereditary non-adenomatous large intestine cancer), 10 cases of large intestine adenomatous tissues (including familial large intestine adenomatosis), 85 cases of stomach cancer tissues, 225 cases of normal large intestine mucosal tissues, and 85 cases of normal stomach mucosa tissues were used to analyze these six gene promoter regions or 5' region about their respective frequencies and tendencies of methylation to investigate. Meanwhile, primers shown in Table 1 were used to detect methylation of each gene of SFRP1, SFRP2, APC, DCC, MGMT, hMLH1. Primers shown in Table 1 comprise each an oligonucleotide having the sequence shown by SEQ ID NOS: 1 to 18 in the sequence table. Location in gene, base length and Tm value of each primer are also shown in Table 1.

**Table 1**

| Name of gene | Name of primer | Location | Sequence (Number shows sequence number in sequence table) | Base length (BP) | Tm (C) |
|---|---|---|---|---|---|
| *SFRP1* | S1-NF | 47440-47463 | GYGTTTTTTTGTTYGTYGTATTTT (13) | 166 | 60 |
| | S1-MF | 47396-47413 | TCGTACCGTCGTTTTTTC (14) | 120 | |
| | S1-NR | 47298-47319 | AAAACRCAATCCCCAACRTTAC (15) | | |
| *SFRP2* | S2-NF | 155072436-155072457 | GGTTGTTAGTTTTTYGGGGTTT (16) | 159 | 62 |
| | S2-MF | 155072508-155072530 | TCGTTTCGTTTTTTTTCGGTTTC (17) | B7 | |
| | S2-NR | 155072570-155072594 | CAACAACAACRAACCAAAACCCTAC (18) | | |
| *APC* | APC1A-NF | 638- 661 | ATATTTTYGAGGGGTAYGGGGTTA (1) | 148 | 58 |
| | APC1A-MF | 702- 719 | TATTGCGGAGTGCGGGTC (2) | 84 | |
| | APC1A-NR | 785- 762 | ACRAAAATAAAAAACRCCCTAATC (3) | | |
| *DOC* | DCC-NF | 4974547-4974569 | AGGTGGAGAAAGAGGTGGAGGAA (4) | 151 | 60 |
| | OCC-MR | 4974660-4974679 | ACCAAAAATCGCGAACAACG (5) | 133 | |
| | DCC-NR | 4974674-4974697 | TCAACCAACACCTTCRAAACCAAA (6) | | |
| *MGMT* | MGMT3-NF | 1023-1053 | GYGTTTYGGATATGTTGGGAT (7) | 134 | 58 |
| | MGMT3-MF | 1075-1092 | ACGTTCGTAGGTTTTCGC (8) | 82 | |
| | MGMT3-NR | 1133-1156 | AACTCCRCACTCTTCCRAAAACRA (9) | | |
| *hMLH1* | MLH15-NF | 1057-1079 | YGGGTAAGTYGTTTTGAYGTAGA (10) | 149 | 58 |
| | MLM15-MF | 1097-1118 | CGTTCGTCGTTCGTTATATATC (11) | 109 | |
| | MLH15-NR | 1182-1205 | TATACCTAATCTATCRCCRCCTCA (12) | | |

In order to detect methylation of each gene, DNAs were amplified by PCR. 2 µL of solution containing DNAs which had been extracted from the tissue and processed with sodium bisulfite as the templates were used with 10 x PCR buffer solution (Invirogen), 1.5 mM MgCl₂, 0.2 mM each primer, 0.1 mM dNTP, 1 unit of Taq polymerase (Platinum taq polymerase: Invirogen) to prepare a total of 20 µL of PCR reaction solution.
According to amplification reaction condition, the solution was heated at 95°C for 3 min, subjected to totally 36 cycles at each cycle of which it was heated at 95°C for 30 sec, at its own Tm temperature for 30 sec, and at 72°C for 30 sec, and then heated at 72°C for 5 min.

Whether each gene had been methylated or not was checked on large intestine cancer samples from 250 cases and large intestine adenoma samples from 10 cases, and it was shown that one or more methylations in these six types of the promoter region and the 5' region were recognized on 100% of large intestine cancer cases (250/250), and 100% of large intestine adenoma cases (10/10), and further on 100% of stomach cancer cases (85/85) for 85 cases.

Assessment of genes was carried out in such a way that genes in which methylation was recognized in the gene promoter regions or the 5' region was given with one point, while a gene in which no methylation was recognized in the same region was given with zero point. For every sample, points in above-mentioned six types of genes and gene loci were added to give a total, which was defined to be M.I. (Methylation Index). Table 2 shows averages in M.I. and DNAs methylation frequencies for large intestine cancer, large intestine adenoma, stomach cancer, normal large intestine mucosa, and normal stomach mucosa. Meanwhile, analysis of variance was used for test of the average of M.I. in every tissue while P-value shows its level. Further, the value within the bracket following an average of M.I. denotes a 95% confidence interval.

**Table 2**

| | | DNA methylation frequency % (Number of methylation samples) | | | | | |
|---|---|---|---|---|---|---|---|
| Tissue | Average of M.I. | SFRP1 | SFRP2 | DCC | APC | MGMT | hMLH1 |
| Large intestine | | | | | | | |
| | | | | | | | |
| Large intestine cancer (n=250) | 3.59 (3.45 -3.73) | 99.6(249) | 80.8(202) | 73.2 (183) | 44.0 (110) | 40.0(100) | 21.2(53) |
| Large intestine ade no ma. (n=10) | 2.60 (2.00 - 3.20) | 100(10) | 50.0(5) | 0(0) | 70.0(7) | 30.0(3) | 10.0(1) |
| Normal mucosa (n=225) | 1.78 (1.66 - 1.90) | 96.9(218) | 17.8(40) | 8.9(20) | 31.6 (71) | 16,9(38) | 5.8(13) |
| | p<0.0001 | | | | | | |
| | | | | | | | |
| Stomach | | | | | | | |
| | | | | | | | |
| Stomach cancer (n=85) | 3.84 (3.60 - 4.09) | 95.3(81) | 88.2(75) | 52.9 (45) | 92.9(79) | 22.4(19) | 32.9(28) |
| Normal mucosa (n=85) | 2.89 (2.68-3.11) | 94.1(80) | 15.3(13) | 24.7(21) | 91.8 (78) | 52.9 (45) | 10.6(9) |
| | p<0.0001 | | | | | | |

As shown in Table 2, a significant difference in average of M.I. was noted among large intestine cancer, large intestine adenoma and normal large intestine mucosa (p<0.0001). Further, a significant difference in average of M.I. was also noted between stomach cancer and normal stomach mucosa (p<0.0001). In other words, it was confirmed that there was a distinctive difference in average of M.I between neoplasm of a digestive organ and normal mucosa of the digestive organ.
Large intestine cancer, for example, was significantly recognized to be methylated exclusively on each of SFRP2, DCC and MGMT genes in comparison to normal mucosa. Large intestine adenoma was significantly recognized to be methylated on each of SFRP2, APC and MGMT genes. Further, stomach cancer was significantly recognized to be methylated on each of SFRP2, DCC and hMLH1 genes. From the results thus obtained, it was suggested that feces sample could be used to detect methylation in these six types of the gene promoter region or the 5' region, thereby to determine M.I. value which would anticipate neoplasm in a digestive organ.

From the results obtained by preliminary investigation, it was suggested that theoretically, a total of methylation values on a plurality of the gene promoter regions or the 5' regions would be different between neoplasm and normal tissue. Similar to these results, it was investigated whether or not a gene contained in feces could be used to detect DNA methylation, thereby to examine neoplasm in a digestive organ.

### (Example 3) Relationship between a total of methylation values on gene promoter region or 5' region, and pathological diagnosis

From 60 patients, who were scheduled to undertake colonoscopy accompanied with the informed consent, feces were collected as biological samples before examination. The feces samples thus collected were supplied with glycogen and processed with sodium bisulfite according to the method as stated in Example 1. 2 µL of the solutions processed with sodium bisulfite were used to detect methylation in the promoter region and/or the 5' region of each of SFRP1, SFRP2, DCC, APC, MGMT, hMLH1 genes by the same method as used in the preliminary investigation.

In terms of methylation status detected on genes contained in feces, and endoscopy examination on colon and upper gastrointestinal tract, the results of 60 cases are shown in Table 3. As for their gender, F denotes female and M denotes male. FOBT stands for fecal occult-blood test, (+) shows fecal occult-blood test positive, (-) shows fecal occult-blood test negative, and N.D. shows a case where whether or not fecal occult-blood test was performed can not be confirmed. As for methylation status, a case with methylation recognized in gene promoter region or 5' region is represented by one, and a case with no methylation recognized is represented by zero. A case with no nucleic acid amplification reaction recognized is shown by N.A. (Not Amplified). As for M.I., a case with methylation recognized in each gene promoter regions and/or 5' region is given with one point to add, and a case with no methylation is shown by N.A. and excluded from addition (i.e. , same to zero point).

**Table 3**

| Sample No. | Gen der | Age | FOBT | Result by endoscopy of upper gastrointestinal tract/pathological diagnosis | Result by endoscopy of large intestine/pathological diagnosis | Methylation status | | | | | | MI. |
|---|---|---|---|---|---|---|---|---|---|---|---|---|
| | | | | | | SFRP1 | SFRP2 | DCC | APC | MGMT | hMLH1 | |
| 53 | F | 89 | + | | Large intestine cancer (T)/Adenocarcinoma | 0 | 1 | 0 | 1 | NA. | NA. | 2 |
| 66 | M | 87 | N.D. | | Large intestine cancer (S)/Adenocarcinoma | 1 | 1 | 1 | 1 | 0 | 1 | 5 |
| 67 | M | 87 | N.D. | | Large intestine cancer (S)/Adenocarcinoma | 1 | 1 | 1 | 1 | 1 | 0 | 5 |
| 90 | F | 61 | N.D. | | Large intestine cancer (S)/Adenocarcinoma | 0 | 1 | 0 | 1 | 0 | MA. | 2 |
| 70 | M | 71 | N.D. | | Large intestine cancer (C)/Adenocarcinoma | 1 | 1 | 0 | N.A. | N.A. | 0 | 2 |
| 12 | M | 67 | + | | Large intestine cancer (A)/Adenoc arcinoma | 1 | 1 | | 1 | N.A. | 0 | 4 |
| 68 | F | 57 | N.D. | | Rectal cancer/Adenocar cinoma | 1 | 1 | 1 | 1 | 1 | 1 | 6 |
| 65 | M | 76 | N.D. | | Rectal cancer/Adenoc or cinoma | 1 | 1 | 1 | 1 | 0 | 0 | 4 |
| 69 | F | 75 | N.D. | | Rectal cancer/Adenocarcinoma | 1 | 1 | 1 | 1 | 0 | 0 | 4 |
| 88 | F | 50 | N.D. | | Rectal cancer/Adenocarcinoma | 1 | 1 | 1 | 0 | 0 | 0 | 3 |
| 50 | F | 79 | + | | Rectal cancer/Adenocarcinoma | 1 | 0 | N.A. | 1 | N.A. | NA. | 2 |
| 91 | F | 67 | N.D. | | Rectal cancer/Adenocarcinoma | 0 | 1 | 0 | N.A. | 1 | NA. | 2 |
| 16 | M | 68 | + | Stomach cancer Adenocarcinoma | Large intestine polyp(D)/Tubular Adenoma | 1 | 1 | 1 | 1 | 0 | 0 | 4 |
| 37 | F | 79 | + | Stomach cancer Adenocarcinoma | No aberration | 1 | 1 | 1 | 1 | 0 | 0 | 4 |
| 43 | F | 82 | N.D. | Duodoum polyp/Carcinoid | No aberration | 1 | 1 | 0 | 1 | NA. | 1 | 4 |
| 33 | M | 73 | N.D. | Chronic gastritis/Suggestion of malignency(-) | Large intestine polyp (T)/Tubular Adenoma | 1 | 1 | 1 | 1 | 1 | NA. | 5 |
| 49 | M | 77 | + | Chronic gastritis/Suggestion of malignency) | Large intestine polyp (T)/Tubular Adenoma | 0 | 1 | 0 | 1 | NA. | 0 | 2 |
| 51 | M | 65 | N.D. | | Large intestine polyp (T)/Tubular Adenoma | 1 | 1 | 0 | 1 | N.A. | 1 | 4 |
| 40 | M | 81 | + | Chronic gastritis/Suggestion of malignency(-) | Large intestine polyp(S)/Tubular Adenoma | 1 | 1 | 0 | 1 | 0 | N.A. | 3 |
| 28 | M | 48 | + | Chronic gastritis/Suggestion of malignency(-) | Large intestine polyp(S)/Tubular Adenoma | 0 | 1 | 0 | 0 | 0 | 0 | 1 |
| 32 | M | 61 | N.D. | No aberration | Large intestine polyp(S)/Tubular Adenoma | 1 | 1 | 0 | 1 | N.A. | N.A. | 3 |
| 4 | M | 70 | N.D. | | Large intestine polyp(S)/Tubular Adenoma | 1 | N A. | N A. | 1 | NA. | 1 | 3 |
| 15 | F | 66 | N.D. | | Large intestine polyp(S)/Tubular Adenoma | 1 | N A. | 0 | 1 | NA. | 0 | 2 |
| 78 | M | 20 | N.D. | | Large intestine polyp(S)Tubular Adenoma | 0 | 1 | 0 | 0 | 0 | 0 | 1 |
| 62 | M | 72 | + | | Large intestine polyp(A)/Tubular Adenoma | 0 | 1 | 0 | 0 | 0 | 0 | 1 |
| 60 | M | 72 | N.D. | Chronic gastritis/Suggestion of malignency(-) | Rectum polyp/Tubular Adenoma | 1 | 0 | 0 | 1 | 0 | NA. | 2 |
| 31 | F | 64 | N.D. | | Rectum polyp/Tubular Adenoma | 1 | 1 | 1 | 1 | 0 | 0 | 4 |
| 17 | F | 63 | + | | Rectum polyp/Tubular Adenoma | 0 | 1 | 1 | 0 | NA. | 0 | 2 |
| 47 | F | 82 | N.D. | Gastric polyp/fundic gland polyp | Diverticuta of large intestin | 1 | 0 | 1 | 0 | NA. | NA | 2 |
| 6 | M | 79 | N.D. | Gastric polyp/hyperplastic polyp | No aberration | 1 | 1 | 0 | 0 | NA. | 0 | 2 |
| 61 | F | 76 | N.D. | Gastric ulcer/Suggestion of malignency(-) | Diverticula of large intestin | 1 | 1 | NA. | NA. | 1 | 0 | 3 |
| 57 | M | 72 | N.D | Gastric ulcer/Suggestion of malignency(-) | No aberration | 0 | 1 | 0 | 1 | NA. | NA. | 2 |
| 38 | M | 42 | N.D. | Gastric ulcer/Suggestion of malignency(-) | No aberration | 0 | 1 | 0 | 1 | NA. | NA. | 2 |
| 7 | F | 53 | N.D. | Chronic gastritis/Suggestion of malignency(-) | Ischemic colitis | 1 | 1 | 1 | 0 | 0 | 0 | 3 |
| 29 | M | 72 | + | | Ischemic colitis | NA. | 1 | 0 | 0 | NA. | 0 | 1 |
| 9 | F | 73 | N.D. | | False melanosis | 1 | 1 | 1 | 0 | NA. | 0 | 3 |
| 10 | M | 64 | N.D. | | Ulcerative colitis | 0 | 1 | NA. | NA. | NA. | NA. | 1 |
| 45 | F | 86 | + | Chronic gastritis/Suggestion of malignency(-) | Diverticula of large intestin | 1 | 1 | 0 | 0 | NA. | 0 | 2 |
| 20 | F | 64 | + | | Diverticula of large intestin | 1 | 1 | 0 | 0 | NA. | 1 | 3 |
| 42 | M | 47 | N.D. | | Diverticulta of large intestin | 1 | 1 | 0 | NA. | NA. | NA. | 2 |
| 52 | F | 63 | + | | Diverticula of large intestin | 0 | 1 | NA. | 1 | NA. | 0 | 2 |
| 58 | F | 65 | N.D. | | Diverticula of large intestin | 0 | 1 | NA. | NA. | 0 | NA. | 1 |
| 19 | F | 71 | + | | Diverticula of large intestin | NA. | NA. | NA. | NA. | NA. | NA. | 0 |
| 63 | F | 82 | - | Chronic gastritis/Suggestion of malignency(-) | No aberration | 0 | 1 | 0 | 0 | 0 | 1 | 2 |
| 41 | F | 76 | + | Chronic gastritis/Suggestion of malignency(-) | No aberration | 0 | 1 | 0 | NA. | NA. | 0 | 1 |
| 2 | M | 65 | N.D. | Chronic gastritis/Suggestion of malignency(-) | No aberration | NA. | 1 | 0 | 0 | 0 | 0 | 1 |
| 27 | F | 73 | N.D. | Chronic gastritis/Suggestion of malignency(-) | Internal hemorrhoid | 1 | 1 | 1 | 1 | NA. | NA. | 4 |
| 56 | F | 45 | N.D. | | Internal hemorrhoid | 1 | NA. | NA. | NA. | NA. | 0 | 1 |
| 46 | F | 76 | + | | No aberration | NA. | 0 | 0 | 0 | NA. | 0 | 0 |
| 11 | F | 71 | + | | No aberration | 1 | 1 | 1 | 1 | NA. | 1 | 5 |
| 35 | F | 73 | N.D. | | No aberration | 1 | 1 | 1 | 1 | NA. | NA. | 4 |
| 30 | M | 61 | N.D. | | No aberration | 1 | 1 | 0 | 1 | 0 | 0 | 3 |
| 55 | F | 62 | N.D. | | No aberration | NA. | 1 | 0 | 1 | NA. | 0 | 2 |
| 8 | F | 66 | N.D. | | No aberration | 1 | 0 | 0 | NA. | NA. | 0 | 1 |
| 36 | F | 71 | N.D. | | No aberration | 1 | NA. | NA. | 0 | NA. | NA. | 1 |
| 59 | F | 70 | + | | No aberration | 1 | 0 | 0 | NA. | 0 | 0 | 1 |
| 39 | M | 62 | - | | No aberration | NA. | NA. | 0 | NA. | NA | NA. | 0 |
| 44 | M | 53 | N.D. | | No aberration | 0 | NA. | NA. | 0 | NA. | NA. | 0 |
| 54 | M | 44 | + | | No aberration | NA. | 0 | 0 | NA. | NA. | 0 | 0 |
| 64 | M | 81 | N.D. | | No aberration | NA. | 0 | NA. | NA. | NA. | NA. | 0 |

60 cases shown in Table 3 are classified into three groups; malignant neoplasm group (including stomach adenocarcinoma, large intestine adenocarcinoma, duodenum carcinoid), benign neoplasm group (including stomach fundic gland polyp, stomach hyperplastic polyp, large intestine tubular adenoma), and W.N.L group (cases with no neoplasm (cancer or adenoma) recognized). Table 4 shows a relationship of these three groups with methylation frequency on promoter region or 5' region in each of APC, DCC, MGMT, hMLH1, SFRP1, and SFRP2 genes, and average of M.I. For P-value, analysis of variance was used for comparison of average of M.I. among three groups. Further, analysis of variance was used for comparison of average of M.I. among three groups. Further, Pearson's test was used for comparison in methylation frequency of each gene promoter region or 5' region among three groups.

**Table 4**

| | | | DNA methylation frequency % (Number of cases for methylated/unmethylated/not amplified) | | | | | |
|---|---|---|---|---|---|---|---|---|
| Endoscopy/ pathological diagnosis | Number of cases | M.1. average ± standard deviation | SFRP1 | SFRP2 | DCC | APC | MGMT | hMLH1 |
| Malignant neoplasm | 15 | 3.53±1.30 | 93.3 | 80.0 | 60.0 | 80.0 | 20.0 | 20.0) |
| | | | (14/1/0) | (12/3/0) | (9/5/1) | (12/1/2) | (3/7/5) | (3/8/4) |
| | | | | | | | | |
| Benign neoplasm | 15 | 2.47±1.19 | 73.3 | 66.7 | 26.7 | 60.0 | 6.7 | 13.3 |
| | | | (11/2/2) | (10/5/0) | (42/10/1) | (9/6/0) | (1/6/8) | (2/8/5) |
| | | | | | | | | |
| W.N.L | 30 | 1.70±1.34 | 66.7 | 46.7 | 16.7 | 26.7 | 3.3 | 10.0 |
| | | | (20/5/5) | (14/8/8) | (5/16/9) | (8/10/12) | (1/6/23) | (3/15/12) |
| | | | | | | | | |
| P-value | | 0.0002 | 0.3882 | 0.0328 | 0.0144 | 0.0015 | 0.0527 | 0.8603 |

As a result, the target genes contained in feces sample could be detected to have their respective methylation values, all of which were totalized to give an average. The average was assigned with a significantly recognizable difference among a case group with malignant neoplasm, a case group with benign neoplasm, and a case group with no neoplasm (W.N.L.). In particular, the malignant neoplasm case group was recognized to have a significant difference in SFRP2, DCC, MGMT and APC genes to discriminate from the case group with no neoplasm, and the benign neoplasm case group was recognized to have a significant difference in SFRP2 and APC genes to discriminate from the case group with no neoplasm. These results exhibited the same tendency as in the preliminary investigation.

Methylation in the SFRP2 promoter region or the 5' region allowed detection of Neoplasm (cancer and adenoma), and methylation in the 1A promoter region of APC gene also exhibited the same tendency. It was revealed that methylation in the DDC Promoter region or the 5' region allowed primarily detection of malignant neoplasm, and methylation in the MGMT promoter region also was recognized to exhibit the same tendency.

In this embodiment, methylation in the promoter region and/or the 5' region of each gene was detected non-quantitatively. Taking account of results shown in Table 4, and assuming that the case having an M.I. value of two or more was assigned to an examination positive group (i.e., case group suspected to have neoplasm in the digestive organs), while the case having an M.I. value of one or zero was designated assigned to an examination negative group, sensitivity and specificity in this examination method were calculated. Results of the calculation are shown in Table 5. Meanwhile, the P-value shows a value of Fisher's exact test.

**Table 5**

| M.I. value | Neoplasm (n=30) % [Number (Malignant/Benign)] | W.N.L (n=30) % [Number] | P-value |
|---|---|---|---|
| 2 or more | 90.0 [27 (15/12)] | 46.7 [14] | 0.0006 |
| Less | 10.0 [3 (0/3)] | 53.3 [16] | |

As shown in Table 5, the examination method could detect neoplasm present in digestive organs at a sensitivity of 90.0% and at a specificity of 53.3%. Further, it was revealed that the method could detect 100% of malignant neoplasm.

### (Comparison example 1) Confirmation by fecal occult blood

60 cases shown in above-mentioned preliminary investigation were subjected to fecal occult-blood testing, and 20 cases of them, who were shown to be positive patients, were checked by colonoscopy to determine whether they had had neoplasm or not. The results are shown in Table 6. W.N.L. is a case with no neoplasm (cancer or adenoma) recognized. As mentioned, more than half of the cases who were positive for fecal occult-blood testing were recognized to have no neoplasm.

**Table 6**

| Pathological diagnosis | Fecal occult-blood reaction (+) |
|---|---|
| Malignant neoplasm | 5/15 (33.3%) |
| Benign neoplasm | 5/15 (33.3%) |
| W.N.L | 10/30 (33.3%) |

From the results mentioned above, it is considered that fecal occult-blood reaction can not almost judge a neoplasm. In the meantime, it is suggested that the examination method according to the present invention can examine neoplasm accurately and simply, because the method detects methylation on a plurality of gene promoter regions or 5' region and adds their respective methylation values to give a total (M.I.).

### Industrial Applicability

As described above, the pretreatment method according to the present disclosure which did not use protein denaturing agent, could modify unmethylated cytosine contained in genes to convert into uracil in a succeeding step.
Further, it was confirmed that biological samples, preferably feces were used to detect methylation in SFRP2 gene, DCC gene and MGMT gene and then determined the total of their respective methylation values, allowing identification of neoplasm (cancer or adenoma) present in a digestive organ. Further, it was suggested that methylation in APC gene and/or hMLH1 gene also was detected to determine the total of their respective methylation values, allowing more accurate examination of large intestine cancer.
Feces samples shown in the Example were investigated to identify methylation in the promoter region and/or the 5' region of the above-mentioned gene. As a result, it was revealed that feces could be used to examine large intestine cancer and/or benign neoplasm.
In this way, it has been confirmed that the pretreatment method according to the present disclosure is practically applicable, because it uses simply feces, a noninvasive DNA material.
In addition, the present invention is not only useful for diagnosis of various disorders, but also applicable for diagnosis of various disorders such as large intestine cancer in a population of normal subjects, because it can deal with lots of samples. The method of the present invention can predict, to some extent, neoplasm in digestive organs. Therefore, the method also can save a patient from an unnecessary burden. For example, only a patient, who is judged to need a more detailed check by the examination method of the present invention, may be subjected to endoscopy.

## Claims

1. A method for detecting methylation in genes and/or gene loci, comprising the following steps:
(a) supplying a biological sample with glycogen and without protein denaturing agent, wherein the biological sample is selected from the group of blood, urine, serum, and feces, wherein the protein denaturing agent is urea, guanidine hydrochloric acid, guanidine sulfate, or guanidine thiocyanate, and
(b) contacting the sample obtained from step (a) with a bisulfite to convert unmethylated cytosine into uracil in genes and/or gene loci contained in the biological sample, thereby to detect cytosine which has not been converted into uracil.

2. A method for detecting methylation in genes and/ or gene loci, comprising the following steps:
(a) supplying a biological sample with glycogen and without protein denaturing agent, wherein the protein denaturing agent is urea, guanidine hydrochloric acid, guanidine sulfate, or guanidine thiocyanate,
(b) heating the biological sample, and
(c) contacting the sample obtained from step (b) with a bisulfite to convert unmethylated cytosine into uracil in genes and/or gene loci contained in the biological sample, thereby to detect cytosine which has not been converted into uracil.

3. A method for examining a neoplasm comprising the following steps:
- supplying a biological sample with glycogen and without protein denaturing agent, wherein the biological sample is selected from the group of blood, urine, serum, and feces, wherein the protein denaturing agent is urea, guanidine hydrochloric acid, guanidine sulfate, or guanidine thiocyanate, and
- carrying out a method for detecting methylation in genes and/or gene loci contained in the biological sample, said method comprising contacting the sample obtained in the preceding step with a bisulfite to convert unmethylated cytosine into uracil in genes and/or gene loci contained in the biological sample, thereby to detect cytosine which has not been converted into uracil.

4. A method for examining a neoplasm comprising the following steps:
- supplying a biological sample with glycogen and without protein denaturing agent, wherein the protein denaturing agent is urea, guanidine hydrochloric acid, guanidine sulfate, or guanidine thiocyanate,
- heating the biological sample, and
- carrying out a method for detecting methylation in genes and/or gene loci contained in the biological sample said method comprising contacting the sample obtained in the preceding step with a bisulfite to convert unmethylated cytosine into uracil in genes and/or gene loci contained in the biological sample, thereby to detect cytosine which has not been converted into uracil.

5. The method for examining a neoplasm according to Claim 3 or 4, wherein the method for detecting methylation comprises detecting methylation in genes and/or gene loci of the SFRP2 gene, the DCC gene and the MGMT gene contained in a biological sample to calculate the total of their respective methylation values.

6. The method for examining a neoplasm according to Claim 5, wherein the method for detecting methylation comprises detecting further methylation in the APC gene and/or the hMLH1 gene to calculate the total of their respective methylation values.

## Patentansprüche

1. Verfahren zum Nachweisen der Methylierung in Genen und/oder Genloci, das die folgenden Schritte umfasst:
(a) Bereitstellen einer biologischen Probe mit Glycogen und ohne proteindenaturierendem Agens, wobei die biologische Probe ausgewählt ist aus der Gruppe aus Blut, Urin, Serum und Faeces, wobei das proteindenaturierende Agens Harnstoff, Guanidiniumhydrochlorid, Guanidiniumsulfat oder Guanidiniumthiocyanat ist,
und
(b) In-Kontakt-Bringen der aus Schritt (a) erhaltenen Probe mit einem Bisulfit, um unmethyliertes Cytosin in Genen und/oder Genloci, die in der biologischen Probe enthalten sind, in Uracil umzuwandeln, um dadurch Cytosin nachzuweisen, das nicht in Uracil umgewandelt worden ist.

2. Verfahren zum Nachweisen von Methylierung in Genen und/oder Genloci, das die folgenden Schritte umfasst:
(a) Bereitstellen einer biologischen Probe mit Glycogen und ohne proteindenaturierendem Agens, wobei das proteindenaturierende Agens Harnstoff, Guanidiniumhydrochlorid, Guanidiniumsulfat oder Guanidiniumthiocyanat ist,
(b) Erwärmen der biologischen Probe, und
(c) In-Kontakt-Bringen der aus Schritt (b) erhaltenen Probe mit einem Bisulfit, um unmethyliertes Cytosin in Genen und/oder Genloci, die in der biologischen Probe enthalten sind, in Uracil umzuwandeln, um dadurch Cytosin nachzuweisen, das nicht in Uracil umgewandelt worden ist.

3. Verfahren zum Untersuchen eines Neoplasmas, das die folgenden Schritte umfasst:
- Bereitstellen einer biologischen Probe mit Glycogen und ohne proteindenaturierendem Agens, wobei die biologische Probe ausgewählt ist aus der Gruppe aus Blut, Urin, Serum und Faeces, wobei das proteindenaturierende Agens Harnstoff, Guanidiniumhydrochlorid, Guanidiniumsulfat oder Guanidiniumthiocyanat ist, und
- Durchführen eines Verfahrens zum Nachweisen von Methylierung in Genen und/oder Genloci, die in der biologischen Probe enthalten sind, wobei das Verfahren umfasst: das In-Kontakt-Bringen der in dem vorangegangenen Schritt erhaltenen Probe mit einem Bisulfit, um unmethyliertes Cytosin in Genen und/oder Genloci, die in der biologischen Probe enthalten sind, in Uracil umzuwandeln, um dadurch Cytosin nachzuweisen, das nicht in Uracil umgewandelt worden ist.

4. Verfahren zum Untersuchen eines Neoplasmas, das die folgenden Schritte umfasst:
- Bereitstellen einer biologischen Probe mit Glycogen und ohne proteindenaturierendem Agens, wobei das proteindenaturierende Agens Harnstoff, Guanidiniumhydrochlorid, Guanidiniumsulfat oder Guanidiniumthiocyanat ist,
- Erwärmen der biologischen Probe, und
- Durchführen eines Verfahrens zum Nachweisen von Methylierung in Genen und/oder Genloci, die in der biologischen Probe enthalten sind, wobei das Verfahren umfasst: das In-Kontakt-Bringen der in dem vorangegangenen Schritt erhaltenen Probe mit einem Bisulfit, um unmethyliertes Cytosin in Genen und/oder Genloci, die in der biologischen Probe enthalten sind, in Uracil umzuwandeln, um dadurch Cytosin nachzuweisen, das nicht in Uracil umgewandelt worden ist.

5. Verfahren zum Untersuchen eines Neoplasmas gemäß Anspruch 3 oder 4, wobei das Verfahren zum Nachweisen von Methylierung umfasst: das Nachweisen von Methylierung in Genen und/oder Genloci des SFRP2-Gens, des DCC-Gens und des MGMT-Gens, die in einer biologischen Probe enthalten sind, um die Gesamtheit ihrer jeweiligen Methylierungswerte zu berechnen.

6. Verfahren zum Untersuchen eines Neoplasmas gemäß Anspruch 5, wobei das Verfahren zum Nachweisen von Methylierung umfasst: das Nachweisen weiterer Methylierung in dem APC-Gen und/oder dem hMLH1-Gen, um die Gesamtheit ihrer jeweiligen Methylierungswerte zu berechnen.

## Revendications

1. Procédé pour la détection de la méthylation dans des gènes et/ ou des loci de gènes, comprenant les étapes suivantes:
(a) fourniture d'un échantillon biologique avec du glycogène et sans agent dénaturant les protéines, tandis que l'échantillon biologique est choisi dans le groupe consistant en sang, urine, sérum et matières fécales, tandis que l'agent dénaturant les protéines est l'urée, l'acide guanidine chlorhydrique, le sulfate de guanidine, ou le thiocyanate de guanidine, et
(b) mise en contact de l'échantillon obtenu à partir de l'étape (a) avec un bisulfite pour convertir la cytosine non-méthylée en uracile dans les gènes et/ou les loci de gènes contenus dans l'échantillon biologique, pour détecter ainsi la cytosine qui n'a pas été convertie en uracile.

2. Procédé pour la détection de la méthylation dans des gènes et/ ou des loci de gènes, comprenant les étapes suivantes:
(a) fourniture d'un échantillon biologique avec du glycogène et sans agent dénaturant les protéines, tandis que l'agent dénaturant les protéines est l'urée, l'acide guanidine chlorhydrique, le sulfate de guanidine, ou le thiocyanate de guanidine,
(b) chauffage de l'échantillon biologique, et
(c) mise en contact de l'échantillon obtenu à partir de l'étape (b) avec un bisulfite pour convertir la cytosine non-méthylée en uracile dans les gènes et/ou les loci de gènes contenus dans l'échantillon biologique, pour détecter ainsi la cytosine qui n'a pas été convertie en uracile.

3. Procédé pour l'examen d'un néoplasme, comprenant les étapes suivantes:
- fourniture d'un échantillon biologique avec du glycogène et sans agent dénaturant les protéines, tandis que l'échantillon biologique est choisi dans le groupe consistant en sang, urine, sérum et matières fécales, tandis que l'agent dénaturant les protéines est l'urée, l'acide guanidine chlorhydrique, le sulfate de guanidine, ou le thiocyanate de guanidine, et
- mise en oeuvre d'un procédé pour la détection de la méthylation dans les gènes et/ou les loci de gènes contenus dans l'échantillon biologique, ledit procédé comprenant la mise en contact de l'échantillon obtenu dans l'étape précédente avec un bisulfite pour convertir la cytosine non-méthylée en uracile dans les gènes et/ou les loci de gènes contenus dans l'échantillon biologique, de manière à détecter la cytosine qui n'a pas été convertie en uracile.

4. Procédé pour l'examen d'un néoplasme, comprenant les étapes suivantes:
- fourniture d'un échantillon biologique avec du glycogène et sans agent dénaturant les protéines, tandis que l'agent dénaturant les protéines est l'urée, l'acide guanidine chlorhydrique, le sulfate de guanidine, ou le thiocyanate de guanidine,
- chauffage de l'échantillon biologique, et
- mise en oeuvre d'un procédé pour la détection de la méthylation dans les gènes et/ou les loci de gènes contenus dans l'échantillon biologique, ledit procédé comprenant la mise en contact de l'échantillon obtenu dans l'étape précédente avec un bisulfite pour convertir la cytosine non-méthylée en uracile dans les gènes et/ou les loci de gènes contenus dans l'échantillon biologique, de manière à détecter la cytosine qui n'a pas été convertie en uracile.

5. Procédé pour l'examen d'un néoplasme selon la revendication 3 ou 4, dans lequel le procédé pour la détection de la méthylation comprend la détection de la méthylation dans des gènes et/ou des loci de gènes du gène SFRP2, du gène DCC et du gène MGMT contenus dans un échantillon biologique pour calculer le total de leurs valeurs de méthylation respectives.

6. Procédé pour l'examen d'un néoplasme selon la revendication 5, dans lequel le procédé pour la détection de la méthylation comprend la détection de méthylation ultérieure dans le gène APC et/ou le gène hMLH1 pour calculer le total de leurs valeurs de méthylation respectives.


### SEQUENCE LISTING

<110> National University corporation okayama University
<120> Method for detecting gene methylation and method for examining neoplasm by methylation detection
<130> 535-5
<140>
   <141> 2007-06-12
<150> JP2004-359471
   <151> 2004-12-13
<150> JP2004-360339
   <151> 2004-12-13
<160> 18
<170> PatentIn version 3.1
<210> 1
   <211> 24
   <212> DNA
   <213> Artificial
<220>
   <223> Designed DNA based on APCgene
<400> 1
   atattttyga ggggtayggg gtta 24
<210> 2
   <211> 18
   <212> DNA
   <213> Artificial
<220>
   <223> Designed DNA based on APCgene
<400> 2
   tattgcggag tgcgggtc 18
<210> 3
   <211> 24
   <212> DNA
   <213> Artificial
<220>
   <223> Designed DNA based on APCgene
<400> 3
   acraaaataa aaaacrccct aatc 24
<210> 4
   <211> 23
   <212> DNA
   <213> Artificial
<220>
   <223> Designed DNA based on DCCgene
<400> 4
   aggtggagaa agaggtggag gaa 23
<210> 5
   <211> 20
   <212> DNA
   <213> Artificial
<220>
   <223> Designed DNA based on DCCgene
<400> 5
   accaaaaatc gcgaacaacg 20
<210> 6
   <211> 24
   <212> DNA
   <213> Artificial
<220>
   <223> Designed DNA based on DCCgene
<400> 6
   tcaaccaaca ccttcraaac caaa 24
<210> 7
   <211> 21
   <212> DNA
   <213> Artificial
<220>
   <223> Designed DNA based on MGMTgene
<400> 7
   gygtttygga tatgttggga t 21
<210> 8
   <211> 18
   <212> DNA
   <213> Artificial
<220>
   <223> Designed DNA based on MGMTgene
<400> 8
   acgttcgtag gttttcgc 18
<210> 9
   <211> 24
   <212> DNA
   <213> Artificial
<220>
   <223> Designed DNA based on MGMTgene
<400> 9
   aactccrcac tcttccraaa acra 24
<210> 10
   <211> 23
   <212> DNA
   <213> Artificial
<220>
   <223> Designed DNA based on hMLH1gene
<400> 10
   ygggtaagty gttttgaygt aga 23
<210> 11
   <211> 22
   <212> DNA
   <213> Artificial
<220>
   <223> Designed DNA based on hMLH1gene
<400> 11
   cgttcgtcgt tcgttatata tc 22
<210> 12
   <211> 24
   <212> DNA
   <213> Artificial
<220>
   <223> Designed DNA based on hMLH1gene
<400> 12
   tatacctaat ctatcrccrc ctca 24
<210> 13
   <211> 24
   <212> DNA
   <213> Artificial
<220>
   <223> Designed DNA based on SFRP1gene
<400> 13
   gygttttttt gttygtygta tttt 24
<210> 14
   <211> 18
   <212> DNA
   <213> Artificial
<220>
   <223> Designed DNA based on SFRP1gene
<400> 14
   tcgtagcgtc gttttttc 18
<210> 15
   <211> 22
   <212> DNA
   <213> Artificial
<220>
   <223> Designed DNA based on SFRP1gene
<400> 15
   aaaacrcaat ccccaacrtt ac 22
<210> 16
   <211> 22
   <212> DNA
   <213> Artificial
<220>
   <223> Designed DNA based on sFRP2gene
<400> 16
   ggttgttagt ttttyggggt tt 22
<210> 17
   <211> 23
   <212> DNA
   <213> Artificial
<220>
   <223> Designed DNA based on sFRP2gene
<400> 17
   tcgtttcgtt ttttttcggt ttc 23
<210> 18
   <211> 25
   <212> DNA
   <213> Artificial
<220>
   <223> Designed DNA based on SFRP2gene
<400> 18
   caacaacaac raaccaaaac cctac 25